# EUROPEAN PATENT SPECIFICATION

(11) **EP 0 607 373 B1**
(45) Date of publication and mention of the grant of the patent: **19.03.1997**
(21) Application number: 93913367.4
(22) Date of filing: 15.06.1993
(51) Int. Cl.: C07C 233/75, A61K 31/165, C07D 213/81, C07D 213/82, C07C 233/29, C07C 235/56, C07D 213/75, C07D 239/42

(54) **TRISUBSTITUTED PHENYL DERIVATIVES AS SELECTIVE PHOSPHODIESTERASE IV INHIBITORS**
TRISUBSTITUIERTE PHENYLDERIVATE ALS SELEKTIVE PHOSPHODIESTERASE IV INHIBITOREN
DERIVES A GROUPE PHENYLE TRISUBSTITUE UTILISES COMME INHIBITEURS SELECTIFS DE LA PHOSPHODIESTERASE IV

(30) Priority: 15.06.1992 GB 9212673; 15.06.1992 GB 9212693
(43) Date of publication of application: 27.07.1994
(73) Proprietor: CELLTECH THERAPEUTICS LIMITED, Slough, Berkshire SL1 4EN (GB)
(72) Inventor: BEELEY, Nigel, Robert, Arnold 16 Cheshire Road, Oxfordshire OX9 3LQ (GB); MILLICAN, Thomas, Andrew 3 Harcourt Close, Maidenhead Berkshire SL6 0DY (GB)
(74) Representative: Skailes, Humphrey John
(86) International application number: GB9301266
(87) International publication number: WO9325517

(56) References cited:
- EP-A- 0 497 564
- WO-A-91/16303
- WO-A-92/07567

## Description

### FIELD OF THE INVENTION

This invention relates to a novel series of trisubstituted phenyl derivatives, to processes for their preparation, to pharmaceutical compositions containing them, and to their use in medicine.

### BACKGROUND TO THE INVENTION

Many hormones and neurotransmitters modulate tissue function by elevating intra-cellular levels of adenosine 3', 5'-cyclic monophosphate (cAMP). The cellular levels of cAMP are regulated by mechanisms which control synthesis and breakdown. The synthesis of cAMP is controlled by adenylyl cyclase which may be directly activated by agents such as forskolin or indirectly activated by the binding of specific agonists to cell surface receptors which are coupled to adenylyl cyclase. The breakdown of cAMP is controlled by a family of phosphodiesterase (PDE) isoenzymes, which also control the breakdown of guanosine 3',5'-cyclic monophosphate (cGMP). To date, seven members of the family have been described (PDE I-VII) the distribution of which varies from tissue to tissue. This suggests that specific inhibitors of PDE isoenzymes could achieve differential elevation of cAMP in different tissues, [for reviews of PDE distribution, structure, function and regulation, see Beavo & Reifsnyder (1990) TIPS, 11: 150-155 and Nicholson et al (1991) TIPS, 12: 19-27].

There is clear evidence that elevation of cAMP in inflammatory leukocytes leads to inhibition of their activation. Furthermore, elevation of cAMP in airway smooth muscle has a spasmolytic effect. In these tissues, PDE IV plays a major role in the hydrolysis of cAMP. It can be expected, therefore, that selective inhibitors of PDE IV would have therapeutic effects in inflammatory diseases such as asthma, by achieving both anti-inflammatory and bronchodilator effects.

The design of PDE IV inhibitors has met with limited success to date, in that many of the potential PDE IV inhibitors which have been synthesised have lacked potency and/or have been capable of inhibiting more than one type of PDE isoenzyme in a non-selective manner. Lack of a selective action has been a particular problem given the widespread role of cAMP in vivo and what is needed are potent selective PDE IV inhibitors with an inhibitory action against PDE IV and little or no action against other PDE isoenzymes.

WO 92/12961 and EP-A 0497564, documents relevant under Article 54(3) and (4) EPC, describe PDE IV inhibitors which are certain N-aryl substituted benzamides.

WO 91/16303 and WO 92/07567 disclose substituted cyclic hydroxamic acids and imidazolidinone derivatives which are also useful as inhibitors of PDE IV.

We have now found a novel series of trisubstituted phenyl derivatives, members of which are potent inhibitors of PDE IV at concentrations at which they have little or no inhibitory action on other PDE isoenzymes. These compounds inhibit the isolated PDE IV enzyme and also elevate cAMP in isolated leukocytes. Certain compounds prevent inflammation in the lungs induced by carrageenan, platelet-activating factor (PAF), interleukin-5 (IL-5) or antigen challenge. These compounds also suppress the hyperresponsiveness of airway smooth muscle seen in inflamed lungs. The compounds of the invention are therefore of use in medicine, especially in the prophylaxis and treatment of asthma.

Thus according to one aspect of the invention, we provide a compound of formula (1) 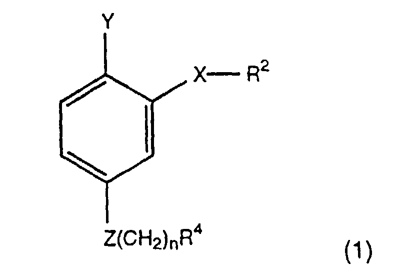 wherein
Y is a group -OR¹ where R¹ is an optionally substituted alkyl group;
R² is an optionally substituted cycloalkyl, or polycycloalkyl group;
Z is a group -N(R³)CO- or -CON(R³)- where R³ is a hydrogen atom or an alkyl, aryl or aralkyl group;
R⁴ is an optionally substituted aryl or optionally substituted heteroaryl group;
X is -O-, -S- -CH₂- or -N(R⁵)-, where R⁵ is a hydrogen atom or an alkyl group;
n is zero or an integer of value 1, 2 or 3; provided that when:
   Z is -CONH-;
   n is zero;
   X is -O-;
   R¹ is an unsubstituted C₁₋₄ alkyl group; and
   R² is an unsubstituted cycloalkyl or polycycloalkyl group;
   then:
   R⁴ is a phenyl, naphthyl or heteroaryl group substituted by one or more substituents selected from C₂₋₆ alkylenedioxy, C₅₋₇ cycloalkoxy, carboxyl (-CO₂H), -CO₂R₇ [where R₇ is a c₆₋₁₂ aryl C₁₋₃ alkyl or C₆₋₁₂ aryl group but is not phenyl or naphthyl] -SO₃H, sulphonylamino (-NHSO₂H), C₁₋₆ alkylsulphonylamino or C₁₋₆ dialkylsulphonylamino groups (this proviso is not valid for the designated states IE and MC); and the salts, solvates and hydrates thereof.

When Y in the compounds of formula (1) is a group OR¹, R¹ may be, for example, an optionally substituted straight or branched alkyl group, for example, a C₁₋₆ alkyl group, e.g. a C₁₋₃ alkyl group, such as a methyl, ethyl, n-propyl, i-propyl, n-butyl, i-butyl, s-butyl, t-butyl, n-pentyl, i-pentyl or n-hexyl group. Optional substituents which may be present on R¹ groups include one or more halogen atoms, e.g. fluorine, chlorine, bromine or iodine atoms.

When R² in the compounds of formula (1) is an optionally substituted cycloalkyl group it may be for example a C₃₋₈ cycloalkyl group such as a cyclobutyl, cyclopentyl or cyclohexyl group, optionally substituted by one, two or three substituents selected from halogen atoms, e.g. fluorine, chlorine, bromine or iodine atoms, C₁₋₆ alkyl e.g. C₁₋₃ alkyl such as methyl or ethyl, hydroxyl or C₁₋₆ alkoxy e.g. C₁₋₃ alkoxy such as methoxy or ethoxy groups. Polycycloalkyl groups represented by the group R² include optionally substituted C₇₋₁₀ polycycloalkyl groups e.g. C₇₋₁₀ bicycloalkyl or C₇₋₁₀ tricycloalkyl groups such as bicyclo[2.2.1] heptyl or indanyl nroups optionally substituted by one, two or three substituents as just described for substituted cycloalkyl groups represented by R².

The group R³ in the compounds of formula (1) may be a hydrogen atom or a straight or brancned C₁₋₆ alkyl group, e.g. a C₁₋₃ alkyl group. Particular groups include methyl, ethyl, n-propyl, i-propyl, n-butyl, i-butyl, s-butyl, or t-butyl groups. Alternatively, R³ may represent a C₆₋₁₂ aryl group such as an optionally substituted phenyl group or a C₆₋₁₂ aryl C₁₋₆ alkyl group, e.g. an optionally substituted phenyl C₁₋₃ alkyl group such as an optionally substituted benzyl or phenethyl group.

When the group R⁴ in compounds of formula (1) is an aryl group, it may be an optionally substituted C₆₋₁₂ aryl group such as an optionally substituted phenyl or 1- or 2-naphthyl group. Heteroaryl groups represented by R⁴ include optionally substituted C₃₋₆ heteroaryl groups containing for example one or two heteroatoms selected from -O- or -S- or groups -N(R⁵)- [where R⁵ is as defined above], such as pyrrolyl, furanyl, oxazolyl, thiazolyl, pyrazoyl, pyridinyl or pyrimidinyl groups. The heteroaryl group may be attached to the remainder of the molecule of formula (1) through any ring carbon or heteroatom as desired.

The aryl or heteroaryl groups represented by R³ and R⁴ in compounds of formula (1) may each optionally be substituted by one, two or more substituents (R⁶) selected from halogen atoms, e.g. fluorine, chlorine, bromine or iodine atoms, or C₁₋₆ alkyl, e.g. methyl or ethyl, C₁₋₆ alkoxy, e.g. methoxy or ethoxy, C₂₋₆ alkylenedioxy, e.g. ethylenedioxy, C₅₋₇ cycloalkoxy, e.g. cyclopentyoxy, halo C₁₋₆ alkyl, e.g. tri-fluoromethyl, C₁₋₆ alkylamino, e.g. methylamino or ethylamino, C₁₋₆ dialkylamino, e.g. dimethylamino or diethylamino, amino (NH₂), nitro, cyano, hydroxyl (OH), carboxyl (CO₂H), -CO₂R⁷ [where R⁷ is a C₁₋₆ alkyl e.g. methyl or ethyl, C₆₋₁₂ aryl C₁₋₃ alkyl, e.g. benzyl or phenethyl or C₆₋₁₂ aryl, e.g. phenyl group], C₁₋₆ alkanoyl e.g. acetyl, sulphonyl (-SO₃H), C₁₋₆ alkylsulphonyl, e.g. methylsulphonyl, aminosulphonyl (-SO₂NH₂), C₁₋₆ alkylaminosulphonyl e.g. methylaminosulphonyl or ethylaminosulphonyl, C₁₋₆ dialkylaminosulphonyl, e.g. dimethylaminosulphonyl or diethylaminosulphonyl, carboxamido (-CONH₂), C₁₋₆ alkylaminocarbonyl, e.g. methylaminocarbonyl or ethylaminocarbonyl, C₁₋₆ dialkylaminocarbonyl, e.g. dimethylaminocarbonyl or diethylaminocarbonyl, sulphonylamino (-NHSO₂H), C₁₋₆ alkylsulphonylamino, e.g. methylsulphonylamino or ethylsulphonylamino, C₁₋₆ dialkylsulphonylamino, e.g. dimethylsulphonylamino or diethylsulphonylamino groups, -NHCOR⁸ (where R⁸ is a C₁₋₆ alkyl group, e.g. a methyl, ethyl, n-propyl, or i-propyl group, or an aryl, e.g. phenyl or aryl C₁₋₃ alkyl, e.g. benzyl, or phenethyl group), -NHCSR⁸, -NHCONH₂, -NHCONH, -NHCONHR⁷, -NHCON(R⁷)₂₄ (where each R⁷ group is the same or different), or C₁₋₆ alkanoylamino C₁₋₆ alkyl, e.g. acetylaminomethyl groups.

It will be appreciated that where two or more R⁶ substitutents are present, these need not necessarily be the same atom and/or groups. The R⁶ substituents may be present at any ring carbon atom away from the atom attached to the rest of the molecule of formula (1). Thus for example, in substituted phenyl groups represented by R³ or R⁴ any substituent(s) may be present at the 2-; 3-; 4-; 5-; 6-; 2,6-; 2,3- or 2,4- positions relative to the ring carbon atom attached to the remainder of the molecule. In another example, when the group R⁴ is a heteroaryl group any carbon atom not attached to the remainder of the molecule may be substituted. One, two or more carbon atoms may have substituents. Thus, in one example, when R⁴ is a substituted 4-pyridinyl group any substituent(s) may be present at the 2-; 3-; 5-; 6-; 2,5- or 3,5 positions relative to the nitrogen atom.

When the group -N(R⁵)- is present in the compounds of formula (1), R⁵ may be a hydrogen atom or a C₁₋₆ alkyl group such as a methyl or ethyl group.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, p-toluenesulphonates, phosphates, sulphates, acetates, trifluoroacetates, propionates, citrates. maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

One particular group of compounds according to the invention has the formula (1) wherein Z is a -N(R³)CO- group and Y, R², R³, R⁴, X and n are as defined for formula (1), and the salts, solvates and hydrates thereof. Particular compounds of this type and those wherein Y is an -OR¹ group, R² is an optionally substituted cycloalkyl or polycycloalkyl group and R¹, R³, R⁴, X and n are as defined for formula (1) and the salts, solvates and hydrates thereof.

Another group of compounds according to the invention has the formula (1) wherein Z is a -CON(R³)- group and Y, R², R³, R⁴, X and n are as defined for formula (1), and the salts, solvates and hydrates thereof. Particular compounds of this type are those wherein Y is an -OR¹ group, R² is an optionally substituted cycloalkyl or polycycloalkyl group and R¹, R³, R⁴ X and n are as defined for formula (1) and the salts, solvates and hydrates thereof.

The following preferences apply alternatively to the compounds of formula (1) when Z is either a -N(R³)CO- or a -CON(R³)- group.

Particularly useful compounds of this type are those where R¹ is an optionally substituted C₁₋₃ alkyl group, particularly an ethyl group, or especially a methyl group.

The group X in compounds of formula (1) may be for example -S-, -CH₂- or -N(R⁵)-, but is preferably -O-.

R² in compounds of formula (1) is preferably an optionally substituted cyclopentyl group.

In one group of compounds of formula (1) n is preferably zero.

In another group of compounds of formula (1) n is an integer of value 1, 2 or 3, and is preferably an integer of value 1.

A useful group of compounds according to the invention has the formula (1) wherein Y is a group -OR¹ where R¹ is an optionally substituted straight or branched C₁₋₃ alkyl group, R² is an optionally substituted C₃₋₈ cycloalkyl group, Z is either -N(R³)CO- or -CON(R³)- where R³ is a hydrogen atom, R⁴ is a C₆₋₁₂aryl or C₃₋₆heteroaryl group, X is -O-, n is zero or an integer of value 1 and the salts, solvates and hydrates thereof. In compounds of this type, n is, in particular, zero.

A particularly useful group of compounds of formula (1) has the formula (2): 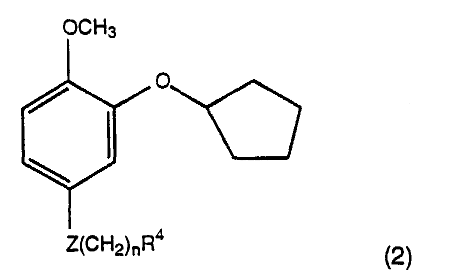 and the salts, solvates and hydrates thereof, wherein Z, n, and R⁴ are as defined for formula (1).

Particular compounds of formula (2) are those wherein n is an integer of value 1, or, especially, is zero.

In the compounds of formulae (1) and (2), Z is preferably - NHCO- or -CONH-.

R⁴ in the compounds of formulae (1) and (2) is preferably an optionally substituted phenyl, or an optionally substituted 2-,3- or, especially, 4-pyridinyl group. Particularly useful substituents include halogen atoms such as fluorine, chlorine, or bromine atoms, and groups selected from nitro, trifluoromethyl, carboxamido, -CO₂CH₃, -CH₃, or -OCH₃.

Particularly useful compounds according to the invention are:
3-Cyclopentyloxy-4-methyloxy-N-(2-Nitrobenzoyl)aniline;
N-(3-Cyclopentyloxy-4-methyloxyphenyl)-4-pyridinecarboxamide;
3-Cyclopentyloxy-4-methyloxy-N-(3-Nitrobenzoyl)aniline;
N-(3-Cyclopentyloxy-4-methyloxyphenyl)-1,2-benzenedicarboxamide;
N-Benzoyl-3-cyclopentyloxy-4-methyloxyaniline;
Methyl 2-[N-(3-Cyclopentyloxy-4-methyloxyphenyl)carbamoyl]benzoate;
N-(3-Cyclopentyloxy-4-methyloxyphenyl)-3,5-dichloro-4 pyridinecarboxamide;
N-Phenyl-3-cyclopentyloxy-4-methyloxybenzamide;
N-(2-Nitrophenyl)-3-cyclopentyloxy-4-methoyloxybenzamide;
N-(3,5-Dichloropyrid-4-yl)-3-cyclopentyloxy-4-methyloxybenzamide;
N-(2,6-Difluorophenyl)-3-cyclopentyloxy-4-methyloxybenzamide;
N-Pyrimidin-4-yl-3-cyclopentyloxy-4-methoxybenzamide;
N-Pyridin-4-yl-3-cyclopentyloxy-4-methyloxybenzamide;
N-Pyrizin-2-yl-3-cyclopentyloxy-4-methyloxybenzamide;
N-Benzyl-3-cyclopentyloxy-4-methyloxybenzamide; and the salts, solvates and hydrates thereof.

Compounds according to the invention are selective and potent inhibitors of PDE IV. The ability of the compounds to act in this way may be simply determined by the tests described in the Examples hereinafter.

The compounds according to the invention are thus of particular use in the prophylaxis and treatment of human diseases where an unwanted inflammatory response or muscular spasm is present and where the elevation of cAMP levels may be expected to prevent or alleviate the inflammation and relax the muscle.

Particular uses to which the compounds of the invention may be put include the prophylaxis and treatment of asthma, especially inflamed lung associated with asthma, or in the treatment of inflammatory airway disease, chronic bronchitis, eosinophilic granuloma, psoriasis and other benign and malignant proliferative skin diseases, endotoxic shock, septic shock, ulcerative colitis, Crohn's disease, reperfusion injury of the myocardium and brain, chronic glomerulonephritis, atopic dermatitis, urticaria, allergic rhinitis, adult respiratory distress syndrome, diabetes insipidus, allergic conjunctivitis and vernal conjunctivitis.

For the prophylaxis or treatment of disease the compounds according to the invention may be administered as pharmaceutical compositions, and according to a further aspect of the invention we provide a pharmaceutical composition which comprises a compound of formula (1) together with one or more pharmaceutically acceptable carriers, excipients or diluents.

Compounds according to the invention may also elevate cAMP in lymphocytes and thereby suppress unwanted lymphocyte activation in immune-based diseases such as rheumatoid arthritis, transplant rejection and graft versus host disease.

Pharmaceutical compositions according to the invention may take a form suitable for oral, buccal, parenteral or nasal administration, or a form suitable for administration by inhalation or insufflation.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds of formula (1) may be formulated for parenteral administration by injection e.g. by bolus injection or continuous infusion. Formulations for injection may be presented in unit dosage form. The compositions for injection may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

In addition to the formulations described above, the compounds of formula (1) may also be formulated as a depot preparation. Such long acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichlorofluoromethane, dichlorotetrafluoroethane, carbon dioxide or other suitable gas.

The compositions may, if desired, be presented in a pack or dispenser device which may contain one or more unit dosage forms containing the active ingredient. The pack or dispense device may be accompanied by instructions for administration.

The quantity of a compound of the invention required for the prophylaxis or treatment of a particular inflammatory condition will vary depending on the compound chosen, and the condition of the patient to be treated. In general, however, daily dosages may range from around 0.01mg/kg to 100mg/kg, e.g. around 1mg/kg to 40mg/kg body weight for oral or buccal administration from around 0.001mg/kg to 50mg/kg body weight for parenteral administration, and around 5mg to around 1000mg for nasal administration or administration by inhalation or insufflation.

The compounds according to the invention may be prepared by the following processes. The symbols Y, R², R³, R⁴ and X when used in the formulae below are to be understood to represent those groups described above in relation to formula (1) unless otherwise indicated.

Thus according to another aspect of the invention, a compound of formula (1) may be prepared either (a) by coupling an amine of formula (3a): 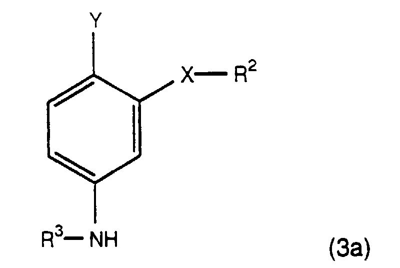 with an acid R⁴(CH₂)ₙCO₂H or an active derivative thereof or (b) by coupling an acid of formula (3b) 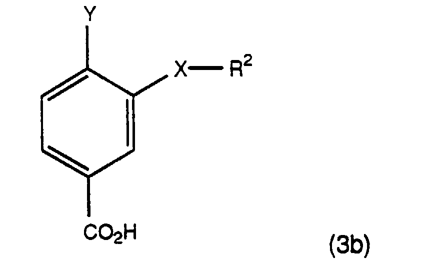 or an active derivative thereof with an amine R⁴(CH₂)ₙNHR³. Active derivatives of acids of formula (3b) or acids R⁴(CH₂)ₙCO₂H include, for example, acid anhydrides, or acid halides, such as acid chlorides.

The coupling reactions may be performed using standard conditions for reactions of this type. Thus for example, the reaction may be carried out in a solvent, for example an inert organic solvent such as an ether, e.g. a cyclic ether such as tetrahydrofuran, an amide, e.g. a substituted amide such as dimethylformamide, or a halogenated hydrocarbon such as dichloromethane, at a low temperature, e.g. -30°C to ambient temperature such as -20°C to 0°C, optionally in the presence of a base, e.g. an organic base such as an amine, e.g. triethylamine or a cyclic amine such as N-methylmorpholine. Where an acid of formula (3b) or R⁴(CH₂)CO₂H is used, the reaction may additionally be performed in the presence of a condensing agent, for example a diimide such as N, N'-dicyclohexylcarbodiimide, advantageously in the presence of a triazole such as 1-hydroxybenzotriazole. Alternatively, the acid may be reacted with a chloroformate, for example ethylchloroformate, prior to reaction with the amine of formula (3).

Intermediate amines of formula (3a) in which R³ is a hydrogen atom may be prepared by hydrogenation of a corresponding nitro compound of formula (4): 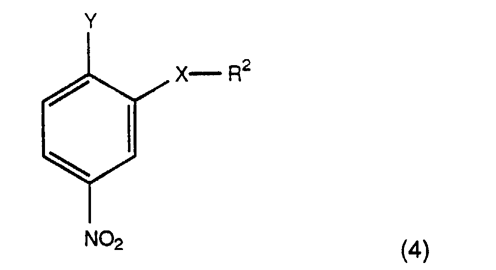 using for example hydrogen and a metal such as palladium or platinum optionally on a support such as carbon, in a solvent such as an alcohol e.g. methanol or ethanol, preferably at an elevated pressure and temperature

Intermediates of formula (3a) wherein R³ is as defined above other than a hydrogen atom may be prepared by reaction of a compound of formula (3a) where R³ is a hydrogen atom with an appropriate halide R³Hal [where Hal is a halogen atom such as a chlorine, bromine or iodine atom] in the presence of a base such as sodium hydride or an alkoxide such as sodium ethoxide, in a solvent such as an alcohol e.g. ethanol, at ambient temperature or above e.g. around 40°C or 50°C.

Intermediates of formula (4) in which X is -O-, -S- or -N(R⁵)- may be prepared by alkylation of a corresponding compound of formula (5): 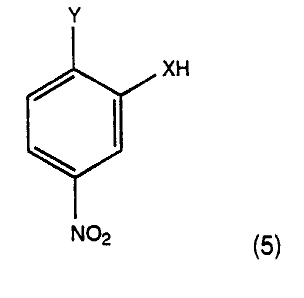 using a reagent R²Hal [where Hal is as just defined]. The reaction conditions may be those just described for the alkylation of intemediates of formula (3a), or, alternatively, where for example the group X is -O- a base such as an alkali metal hydroxide or carbonate, e.g. cesium carbonate may be used in an inert solvent such dimethylformamide at ambient temperature.

Intermediates of formula (4) in which X is -CH₂-, may be prepared by reaction of a compound of formula (6) 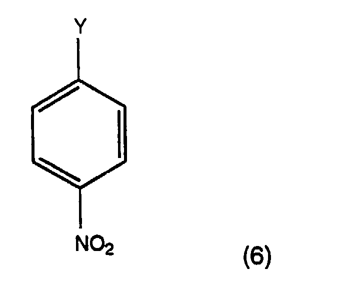 with an acid halide R²COHal in the presence of a catalyst such as aluminium chloride followed by reduction using zinc and an inorganic acid such as hydrochloric acid, or hydrazine and a base such as an alkali metal hydroxide, e.g. sodium hydroxide.

Intermediates of formulae (5) and (6) and the reagents R⁴(CH₂)ₙCO₂H and R⁴Hal are either known compounds or may be prepared from known starting materials by methods analogous to those used for the preparation of the known compounds.

Intermediate acids of formula (3b) may be prepared by hydrolysis of a corresponding ester of formula (7): 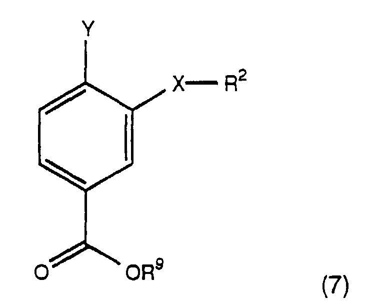 where R⁹ is an alkyl group such as a methyl group, by heating in the presence of a base, for example an alkali metal hydroxide such as lithium hydroxide in a solvent such as an alcohol, e.g. methanol.

Intermediates of formula (7) in which X is -O-, -S- or -N(R⁵)- may be prepared by alkylation of a corresponding compound of formula (8): 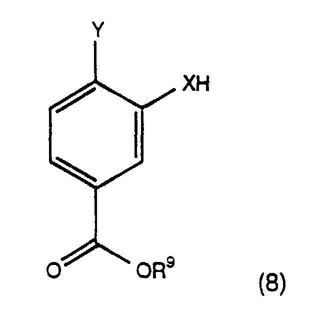 using a reagent R²Hal [where Hal is as just defined] using the reagents and conditions as just described for the preparation of intermediates of formula (5).

Intermediates of formula (7) in which X is -CH₂-, may be prepared by reaction of a compound of formula (9): 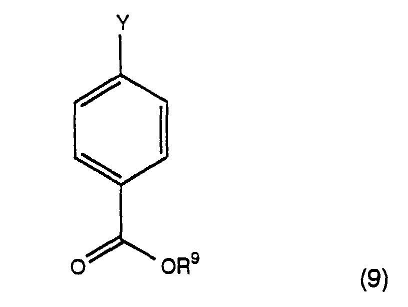 with an acid halide R²COHal as described above for the preparation of compounds of formula (6)

Intermediates of formulae (8) or (9) may be prepared by esterification of the corresponding acids of formulae (10) or (11): 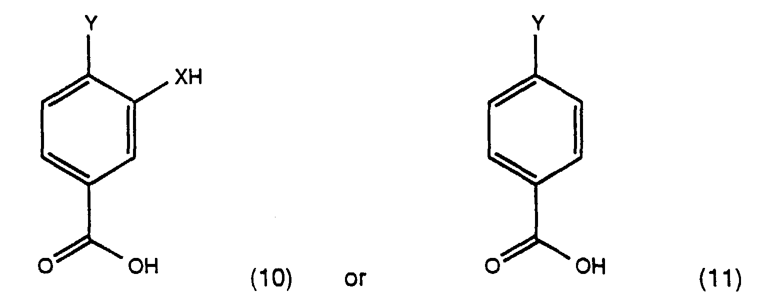 using for example acetyl chloride and an alcohol R⁹OH at an elevated temperature.

Intermediate acids of formulae (10) and (11) and the reagents R⁴(CH₂)ₙNHR³, R²Hal and R⁹OH are either known compounds or may be prepared from known starting materials by methods analogous to those used for the preparation of the known compounds.

Salts of compounds of formula (1) may be prepared by reaction with an appropriate acid or base in a suitable solvent using conventional procedures.

### DESCRIPTION OF SPECIFIC EMBODIMENTS

The following Examples illustrate the invention.

### INTERMEDIATE 1

### 3-Cyclopentyloxy-4-methyloxy-1-nitrobenzene

2-Methyloxy-5-nitrophenol (14.27g, 84.4mmol) was dissolved in dry dimethylformamide (80ml). Cyclopentylbromide (176g, 127ml, 118.2mmol) and cesium carbonate (38.5g, 118.2mmol) were added and the suspension stirred overnight at room temperature. The mixture was poured into saturated Na₂CO₃ (200ml) and extracted with ethyl acetate (700ml). The organic layer was washed with saturated Na₂CO₃ until no yellow colour was detected. The organic layer was then dried (MgSO₄) and concentrated in vacuo to give the title compound as a yellow solid (15.0g).

¹H NMR δ (CDCl₃) 7.85 (1H, dd), 7.72 (1H, d), 6.90 (1H, d), 4.82-4.92 (1H, m), 3.92 (3H, s), 1.60-2.10 (8H, m).

### INTERMEDIATE 2

### 3-Cyclopentyloxy-4-methyloxyaniline

Intermediate 1 was dissolved in degassed methanol and 10% Pd/C (450mg) was added. The mixture was stirred under H₂ gas at room temperature overnight. The catalyst was removed by filtration and the methanol concentrated in vacuo to give the title compound as a dark oil (4.3g).

¹H NMR δ (CDCl₃) 6.70 (1H, d), 6.27 (1H, d), 6.20 (1H, dd), 4.68-4.78 (1H, m), 3.75 (3H, s), 3.42 (1H, br s), 1.50-1.95 (8H, m).

### INTERMEDIATE 3

### 3-Hydroxy-4-methoxy-O-methylbenzoate

To methanol (150ml) at 0°C was added acetyl chloride (5.6g, 5.07ml). After 10 minutes a suspension of 3-Hydroxy-4-methoxybenzoic acid (10g) was added. The reaction mixture was refluxed for 6 hours, cooled and concentrated in vacuo. The residue was dissolved in H₂O, washed with NaHCO₃ (2x100ml), dried MgSO₄ and concentrated to give the title compound as a crystalline solid (9.8g).

¹H NMR δ (CDCl₃) 7.68 (1H, dd), 7.55(1H, d), 6.85 (1H, d), 4.80-4.91 (1H, m), 4.90 (3H, s), 4.88 (3H, s), 1.55-2.08 (8H, m).

### INTERMEDIATE 4

### 3-Cyclopentyloxy-4-methyloxy-O-methylbenzoate

Intermediate 3 was refluxed in dry acetone (20ml) in the presence of K₂CO₃ (1.65g) and cyclopentylbromide (1.77g, 1.6ml) for 48 hours. The mixture was poured into ether/Na₂CO₃. The ether layer was removed and washed with Na₂CO₃ (3x100ml). The organic layer was dried over MgSO₄ and concentrated to give a white solid, recrystallisation of which from H₂O/hexane gave the title compound (1.54g) as a white solid.

¹H NMR δ (CDCl₃) 7.62 (1H, dd), 7.57 (1H, d), 6.85 (1H, d), 4.80-4.90 (1H, m), 3.90 (3H, s), 3.88 (3H, s), 1.55-2.08 (8H, m).

### INTERMEDIATE 5

### 3-Cyclopentyloxy-4-methyloxy benzoic acid

Intermediate 4 (1.18g) was added to CH₂Cl₂/methanol/H₂O (15:5:5, 25ml) and heated to reflux for 2 hours. The reaction mixture was cooled and poured into 1.0m HCI (50ml), then extracted with ethyl acetate (50ml). The organic layer was removed, washed with 1.0M HCI (3x50ml), dried (MgSO₄) and concentrated in vacuo to give the title compound (900mg) as a white solid which was recrystallised twice from ethyl acetate/hexane to give a white solid (450mg).

¹H NMR δ (CDCl₃): 7.75 (1H, dd), 7.60 (1H, d), 6.88 (1H, d), 4.70-4.80 (1H, m), 3.92 (3H, s), 1.55-2.05 (8H, m).

### EXAMPLE 1

### N-Benzoyl-3-cyclopentyloxy-4-methyloxyaniline

To an ice cooled solution of Intermediate 2 (20g, 9.66mmol) in anhydrous pyridine (20ml) was added benzoyl chloride dropwise (2.04g, 1.68ml, 14.5mmol) followed by N,N-dimethylaminopyridine (5mg). The reaction mixture was stirred at room temperature for 3 hours, poured into 1.0M HCl (100ml) and extracted with ethyl acetate. The ethyl acetate layer was washed with 1.0M HCI (2x50ml) and dried over MgSO₄. Concentration in vacuo gave a brownish oil which was recrystallised twice from CH₂Cl₂/hexane to give the title compound as a white solid (1.0g).

¹H NMR δ (CDCl₃) 7.80-7.95 (3H, m), 7.40-7.55 (4H, m), 6.98 (1H, dd),6.82 (1H, d), 4.72-4.82 (1H, m), 3.83 (3H, s), 1.50-2.05 (8H, m).

The following compounds were prepared in a similar manner to the compound of Example 1 using Intermediate 2 and the approprirate chloride:

### EXAMPLE 2

### 3-Cyclopentyloxy-4-methyloxy-N-(3-Nitrobenzoyl)aniline

¹H NMR δ (CDCl₃) 8.70 (1H, br s), 8.36 (1H, d), 8.28 (1H, d), 8.20 (1H, s), 7.68 (1H, t), 7.41 (1H, s), 7.07 (1H, dd), 6.85 (1H, d), 4.70-4.82 (1H, m), 3.85 (3H, s), 1.50-2.05 (8H, m).

### EXAMPLE 3

### 3-Cyclopentyloxy-4-methyloxy-N-(2-Nitrobenzoyl)aniline

¹H NMR δ (CDCl₃ ) 8.10 (1H, d), 7.60-7.80 (3H, m), 7.43 (1H, s), 7.35 (1H, d), 6.90 (1H, dd), 6.85 (1H, d), 4.75-4.85 (1H, m), 3.87 (3H, s), 1.50-2.05 (8H, m).

### EXAMPLE 4

### 3-Cyclopentyloxy-4-methyloxy-N-(4-Nitrobenzoyl)aniline

¹H NMR δ (CDCl₃) 8.35 (2H, d), 8.02 (2H, d), 7.85 (1H, br s), 7.45 (1H, d), 7.05 (1H, dd), 6.87 (1H, d), 4.75-4.85 (1H, m), 3.87 (3H, s), 1.50-2.05 (8H, m).

### EXAMPLE 5

### N-(3-Cyclopentyloxy-4-methyloxyphenyl)-2-pyridinecarboxamide

¹H NMR δ (CDCl₃) 9.95 (1H, br s), 8.58 (1H, dd), 8.28 (1H, dd), 7.90 (1H, m), 7.62 (1H, d), 7.45 (1H, m), 7.12 (1H, dd), 6.88 (1H, d), 4.80-4.90 (1H, m), 3.87 (3H, s), 1.55-2.10 (8H, m).

### EXAMPLE 6

### N-(3-Cyclopentyloxy-4-methyloxyphenyl)-3-pyridinecarboxamide

¹H NMR δ (CDCl₃) 9.08 (1H, d), 8.78 (1H, dd), 8.20 (1H, dd), 8.05 (1H, br s), 7.35-7.45 (2H, m), 7.02 (1H, dd), 6.85 (1H, d), 4.75-4.85 (1H, m), 3.87 (3H, s), 1.55-2.10 (8H, m).

### EXAMPLE 7

### N-(3-Cyclopentyloxy-4-methyloxyphenyl)-4-pyridinecarboxamide

¹H NMR δ (CDCl₃) 8.76 (2H, d), 8.0 (1H, br s), 7.70 (2H, d), 7.45 (1H, d),7.05 (1H, dd), 6.85 (1H, d), 4.75-4.85 (1H, m), 3.88 (3H, s), 1.55-2.05(8H, m).

### EXAMPLE 8

### N-(3-Cyclopentyloxy-4-methyloxyphenyl)-2-phenylacetamide

¹H NMR δ (CDCl₃) 7.24-7.45 (6H, m), 7.02 (1H, br s), 6.68-6.80 (2H, m), 4.70-4.80 (1H, m), 3.80 (3H, s), 3.72 (2H, s), 1.55-2.05 (8H, m).

### EXAMPLE 9

### N-(3-Cyclopentyloxy-4-methyloxyphenyl)-1,2-benzenedicarboxamide

¹H NMR δ (CDCl₃) 8.98 (H, s), 7.60 (1H, dd), 7.48 (1H, dd), 7.35-7.45 (3H, m), 7.05 (1H, dd), 6.78 (2H, br s + d), 5.90 (1H, br s), 4.70-4.80 (1H, m), 3.83 (3H, s), 1.50-2.05 (8H, m).

### EXAMPLE 10

### Methyl-2-[N-(3-Cyclopentyloxy-4-methyloxyphenyl)carbamoyl]benzoate

¹H NMR δ (CDCl₃) 7.92 (H, d), 7.48-7.65 (4H, m), 7.49 (1H, d), 7.0 (1H, dd), 6.83 (1H, d), 4.75-4.87 (1H, m), 3.88 (3H, s), 3.83 (3H, s), 1.5-2.0 (8H, m).

### EXAMPLE 11

### 2-N(3-Cyclopentyloxy-4-methyloxyphenyl)carbamoyl benzoic acid

¹H NMR δ (CD₃OD) 8.02 (1H, d), 7.50-7.65 (3H, m), 7.37 (1H, dd), 7.10 (1H, dd), 6.87 (1H, d), 4.75-4.85 (1H, m), 3.80 (3H, s), 1.55-2.0 (8H, m).

### EXAMPLE 12

### N-(3-Cyclopentyloxy-4-methyloxyphenyl)-N-methyl-2-nitrobenzamide

¹H NMR δ (CDCl₃) 8.10 (1H, d), 7.55-7.75 (3H, m), 7.35 (1H, d), 6.98(1H, dd), 6.83 (1H, d), 4.78-4.85 (1H, m), 3.86 (3H, s), 2.7 (3H, s), 1.50-2.05 (8H, m).

### EXAMPLE 13

### N-(3-Cyclopentyloxy-4-methyloxyphenyl)-3-cyclopentyloxy-4-methyloxybenzamide

¹H NMR δ (CDCl₃) 7.75 (1H, br s), 7.50 (2H, s), 7.38 (1H, dd), 6.8-7.0 (3H, m), 4.75-4.90 (2H, m), 3.90 (3H, s), 3.85 (3H, s), 1.50-2.05 (16H, m).

### EXAMPLE 14

### N(3-Cyclopentyloxy-4-methyloxyphenyl)-N-phenyl-4-pyridinecarboxamide

¹H NMR δ (CDCl₃ 8.50 (2H, d, J = 6Hz), 7.27 (2H, d, J = 6Hz), 7.19-7.37 (5H, m), 6.60-6.75 (3H, m), 4.58 (1H, br s), 3.80 (3H, s), 1.62-1.85 (6H, m), 1.50-1.62 (2H, m).

### EXAMPLE 15

### N-Phenyl-3-cyclopentyloxy-4-methyloxybenzamide

Intermediate 5 (448mg) was dissolved in dry tetrahydrofuran (20ml). N-methylmorpholine (191mg, 209µl) was added and the reaction left 30 minutes to activate. Neat aniline (510mg, 500µl) was added and the reaction stirred at room temperature overnight. The mixture was then poured into 1.0M HCI and ethyl acetate (50ml) was added. The organic layer was separateed, washed with 1.0M HCI (3x50ml), aqueous Na₂CO₃ (3x50ml) and dried (MgSO). Recrystallisation from ethyl acetate/hexane gave the title compound as a white solid (100g).

¹H NMR δ (CDCl₃) 7.77 (1H, br s), 7.64 (2H, d), 7.49 (1H, d), 7.30-7.40 (3H, m), 7.14 (1H, t), 6.88 (1H, d), 4.82-4.92 (1H, m), 3.90 (3H, s), 1.55-2.05 (8H, m).

The following compounds were prepared in a similar method to that described for the preparation of the compound of Example 15, from Intermediate 5 and the appropriate amine:

### EXAMPLE 16

### N-(2-Nitrophenyl)-3-cyclopentyloxy-4-methyloxybenzamide

¹H NMR δ (CDCl₃) 11.34 (1H, br s), 9.01 (1H, d), 8.30 (2H, d), 7.72 (1H, t), 7.55-7.60 (2H, m), 7.22 (2H, t), 7.0 (1H, d), 4.85-4.95 (1H, m), 3.93 (3H, s), 1.55-2.10 (8H, m).

### EXAMPLE 17

### N-Benzyl-3-cyclopentyloxy-4-methyloxybenzamide

¹H NMR δ (CDCl₃) 7.42 (1H, d), 7.20-7.40 (6H, m), 6.85 (1H, d), 6.40 (1H, br s), 4.80-4.92 (1H, m), 4.65 (2H, d), 3.88 (3H, s), 1.48-2.05 (8H, m).

### EXAMPLE 18

### N-(3,5-dichloropyrid-4-yl)-3-cyclopentyloxy-4-methyloxybenzamide

¹H NMR δ (CDCl₃) 8.55 (2H, s), 7.71 (1H, s), 7.48-7.52 (2H, m), 6.93 (1H, d, J = 8Hz), 4.85-4.95 (1H, m), 3.93 (3H, s), 1.60-2.10 (8H, br m).

### EXAMPLE 19

### N-(2,6-Difluorophenyl)-3-cyclopentyloxy-4-methyloxybenzamide

¹H NMR δ (CDCl₃) 6.83-7.60 (7H, m), 4.84 (1H, m), 3.90 (1H, s), 1.55-2.07 (8H, m).

### EXAMPLE 20

### N-Pyrimidin-4-yl-3-cyclopentyloxy-4-methoxybenzamide

¹H NMR δ (CDCl₃) 8.87 (1H, s), 8.67 (1H, d d), 8.55 (1H, br s), 8.30-8.35 (1H, dd), 7.52 (1H, d), 7.4 (1H, dd), (2H, d + dd), 6.9-6.95 (1H, d), 4.82-4.85 (1H, m), 3.91 (3H, s), 1.55-2.0 (8H, m).

### EXAMPLE 21

### N-Pyridin-4-yl-3-cyclopentyloxy-4-methyloxybenzamide

¹H NMR δ (CDCl₃) 8.5-8.7 (2H, dd), 8.02-8.15 (1H, br s), 7.59-7.60 (2H, dd), 7.39-7.40 (1H, dd), 7.47 (1H, s), 6.85-6.91 (1H, d), 4.3-4.4 (1H, m), 3.9 (3H, s), 1.55-2.05 (8H, m).

### EXAMPLE 22

### N-Pyrizin-2-yl-3-cyclopentyloxy-4-methyloxybenzamide

¹H NMR δ (CDCl₃) 9.7 (1H, d), 8.45-8.52 (1H, br s), 8.49 (1H, d), 8.25 (1H, dd), 7.49 (1H, d), 7.4 (1H, dd), 6.9-7.0 (1H, d), 4.85-4.95 (1H, m), 3.95 (3H, s), 0.8-2.2 (8H, m).

The activity and selectivity of compounds according to the invention was demonstrated in the following tests.

### 1. Isolated Enzyme

The potency and selectivity of the compounds of the invention was determined using a battery of distinct PDE isoenzymes as follows:
i. PDE I, rabbit heart
ii. PDE II, rabbit heart
iii. PDE III, rabbit heart
iv. PDE IV, HL60 cells.

The enzymes were purified to kinetic homogeneity using standard chromatographic techniques.

Phosphodiesterase activity was assayed as follows. The reaction was conducted in 150µl of standard mixture containing (final concentrations): 50mM TES-NaOH buffer (pH 7.5), 10mM MgCl₂, 0.1µM [³H]-cAMP and vehicle or various concentrations of the test compounds. The reaction was initiated by addition of enzyme and conducted at 30°C for between 5 to 30 mins. The reaction was terminated by addition of 50µl 2% trifluoroacetic acid containing [¹⁴C]-5'AMP for determining recovery of the product. An aliquot of the sample was then applied to a column of neutral alumina and the [³H]-cAMP eluted with 10ml 0.1 TES-NaOH buffer (pH8). The [³H]-5'-AMP product was eluted with 2ml 2M NaOH into a scintillation vial containing 10ml of scintillation cocktail. Recovery of [³H]-5'AMP was determined using the [¹⁴C]-5'AMP and all assays were conducted in the linear range of the reaction.

Compounds according to the invention were able to inhibit the action of the PDE IV HL60 enzyme at concentrations at which they had little or no effect on the action of each of the other PDE isoenzymes. Thus, compounds of the Examples have approximate Ki values (Ki PDE IV HL60 at (1µM) in the pM-µM range, for example the compounds of Examples 1 and 3 have approximate Ki values of 391nM and 179nM respectively.

### 2. The Elevation of cAMP In Leukocytes

The effect of compounds of the invention on intracellular cAMP was investigated using human neutrophils or guinea pig eosinophils. Human neutrophils were separated from peripheral blood, incubated with dihydrocytochalasin B and the test compound for 10 min and then stimulated with FMLP. Guinea pig eosinophils were harvested by peritoneal lavage of animals previously treated with intra-peritoneal injections of human serum. Eosinophils were separated from the peritoneal exudate and incubated with isoprenaline and test compound. With both cell types, suspensions were centrifuged at the end of the incubation, the cell pellets were resuspended in buffer and boiled for 10 min prior to measurement of cAMP by specific radioimmunoassay (DuPont).

The most potent compounds according to the invention induced a concentration -dependent elevation of cAMP in neutrophils and/or eosinophils at concentrations in the range 1nM to 1µM.

### 3. Suppression of Leukocyte Function

Compounds of the invention were investigated for their effects on superoxide generation and chemotaxis of human neutrophils. Neutrophils were separated from peripheral blood, incubated with dihydrocytochalasin B for superoxide generation only and test compound prior to stimulation with FMLP. The most potent compounds caused a concentration-dependent inhibition of superoxide generation and chemotaxis at concentrations in the range 0.1nM to 2µM.

### 4. Relaxation of Constricted Airway Smooth Muscle in vitro

The effects of compounds of the invention on guinea-pig isolated tracheal smooth muscle were investigated. Isolated tracheal rings were suspended in organ baths and immersed in oxygenated Krebs' solution. The smooth muscle was contracted with sub-maximal concentrations of histamine or carbachol prior to the addition of increasing concentrations of test compound to the organ baths. The most potent compounds caused a concentration-dependent reversal of both histamine and carbachol-induced contractions at concentrations in the range 1nM to 100µM. The compounds were generally more potent in reversing histamine-induced tone than carbachol-induced tone.

### 5. Effects on Cardiac Muscle in vitro

Compounds of the invention have been tested for their effects on isolated cardiac muscle. Right atrial and papillary muscles were dissected out from the hearts of guinea pigs and suspended in organ baths for measuring the rate (chronotropic) of spontaneously beating atria and force (inotropic) of the electrically stimulated papillary muscle. In these preparations, selective PDE IV inhibitors such as rolipram do not have any direct effects whereas selective PDE III inhibitors such as milrinone have positive chronotropic and inotropic effects. The nonspecific PDE inhibitor theophylline, which is used in asthma as a bronchodilator, also causes significant cardiovascular changes such as tachycardia. Selective PDE IV inhibitors have advantage over theophylline, therefore, through reduced cardiovascular side effects. The most potent and selective compounds of the invention had no direct effects on the atrial and papillary muscles in vitro at concentrations up to 100µM but in combination with PDE III inhibitors, these inhibitors showed an enhancement of chronotropic and inotropic activity, typical of selective type IV inhibitors.

### 6. Anti-inflammatory Activity in vivo

Compounds of the invention have been tested in models of experimental pleurisy in the rat induced by carrageenan, platelet-activating factor (PAF), or interleukin-5 (IL-5). The compounds caused a dose-dependent reduction in carrageenan-induced accumulation of inflammatory exudate and total leukocyte numbers at 6h following oral (1-50mg/kg), intravenous (0.1-10mg/kg), or intraperitoneal (0.1-10mg/kg) administration. Compounds of the invention also reduced PAF or IL-5-induced pleural eosinophilia at 24h following oral (1-50mg/kg), intravenous (0.1-10mg/kg) or intraperitoneal (0.1-10mg/kg) dosing.

### 7. Anti-allergic Activity in vivo

Compounds of the invention have been tested for effects on an IgE-mediated allergic pulmonary inflammation induced by inhalation of antigen by sensitised guinea pigs. Guinea pigs were initially sensitised to ovalbumin under mild cyclophosphamide-induced immunosuppression, by intraperitoneal injection of antigen in combinations with aluminium hydroxide and pertussis vaccine. Booster doses of antigen were given two and four weeks later and at six weeks, animals were challenged with aerosolised ovalbumin whilst under cover of an intraperitoneally administered anti-histamine agent (mepyramine). After a further 48h, bronchial alveolar lavages (BAL) were performed and the numbers of eosinophils and other leukocytes in the BAL fluids were counted. The lungs were also removed for histological examination for inflammatory damage. Administration of compounds of the invention (0.1-10mg/kg i.p.), up to three times during the 48h following antigen challenge, lead to a significant reduction in the eosinophilia and the accumulation of other inflammatory leukocytes. There was also less inflammatory damage in the lungs of animals treated with compounds of the invention.

### 8. Effects on Pulmonary Dynamics

Compounds of the invention have been tested for their effects on ozone-induced hyperreactivity of the airways of guinea pigs. Following the inhalation of ozone, guinea pigs become very much more sensitive to the bronchoconstrictor effects of inhaled histamine than naive animals. There is a pronounced shift to the left (10-30 fold) of the dose response curve to histamine and a highly significant increase in the maximum increase in pulmonary resistance. Compounds of the invention administered 1h prior to ozone by the intraperitoneal (0.01-1mg/kg) or oral (0.1-10mg/kg) route caused a dose-dependent inhibition of ozone-induced hyperreactivity.

In general, in our tests above, compounds of the invention have had no observed toxic effects when administered to animals at the doses shown.

## Claims (Claims for the following Contracting State(s): AT, BE, CH, LI, DE, DK, ES, FR, GB, GR, IT, LU, NL, PT, SE)

1. A compound of formula (1) 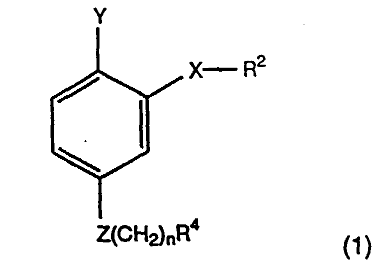 wherein
Y is a group -OR¹ where R¹ is an optionally substituted alkyl group;
R² is an optionally substituted cycloalkyl or polycycloalkyl group;
Z is a group -N(R³)CO- or -CON(R³)- where R³ is a hydrogen atom or an alkyl, aryl or aralkyl group; R⁴ is an optionally substituted aryl or optionally substituted heteroaryl group;
X is -O-, -S-, -CH₂- or -N(R⁵)-, where R⁵ is a hydrogen atom or an alkyl group;
n is zero or an integer of value 1, 2, or 3;
provided that when:
Z is -CONH-;
n is zero;
X is -O-;
R¹ is an unsubstituted C₁₋₄ alkyl group; and
R² is an unsubstituted cycloalkyl or polycycloalkyl group;
then:
R⁴ is a phenyl, naphthyl or heteroaryl group substituted by one or more substituents selected from C₂₋₆ alkylenedioxy, C₅₋₇ cycloalkoxy, carboxyl (-CO₂H), -CO₂R₇ [where R₇ is a C₆₋₁₂ aryl C₁₋₃ alkyl or C₆₋₁₂ aryl group but is not phenyl or naphthyl] -SO₃H, sulphonylamino (-NHSO₂H), C₁₋₆ alkylsulphonylamino or C₁₋₆ dialkylsulphonylamino groups;
and the salts, solvates and hydrates thereof.

2. A compound according to claim 1 wherein Z is a -N(R³)CO- group.

3. A compound according to claim 2 wherein Z is a -NHCO- group.

4. A compound according to claim 1 wherein Z is a -CON(R³)- group.

5. A compound according to claim 4 wherein Z is a -CONH- group.

6. A compound according to any preceding claim where R¹ is an optionally substituted straight or branched C₁₋₃ alkyl group.

7. A compound according to claim 6 wherein R⁷ is a straight or branched C₁₋₃ alkyl group optionally substituted by one or more halogen atoms.

8. A compound according to claim 7 where R¹ is a methyl group.

9. A compound according to any one of claims 1 to 8 where n is zero or an integer of value 1.

10. A compound according to claim 9 where n is zero.

11. A compound according to any one of claims 1 to 10 where R² is an optionally substituted C₃₋₈ cycloalkyl group.

12. A compound according to claim 11 where R² is a cyclobutyl, cyclopentyl or cyclohexyl group optionally substituted by one, two or three halogen atoms, C₁₋₆ alkyl or C₁₋₆ alkoxy groups.

13. A compound according to claim 12 where R² is a cyclopentyl group.

14. A compound a according to any one of claims 1 to 13 wherein R⁴ is an optionally substituted phenyl or pyridinyl group.

15. A compound according to claim 14 wherein R⁴ is an optionally substituted 4-pyridinyl group.

16. A compound according to any of the preceding claims wherein X is -O-.

17. A pharmaceutical composition comprising a compound according to any one of claims 1 to 16 and a pharmaceutically acceptable diluent, carrier or excipient.

18. A process for the preparation of a compound of formula (1) as defined in claim 1 which comprises either (a) coupling an amine of formula (3a) 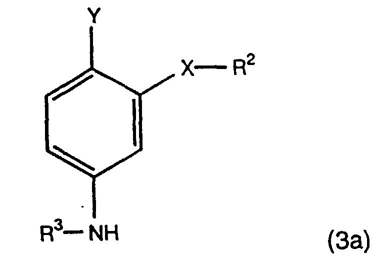 wherein
Y, R², R³ and X are as defined for formula (1), with an acid R⁴(CH₂)ₙCO₂H, where R⁴ and n are as defined for formula (1), or an active derivative thereof or (b) by coupling an acid of formula (3b) 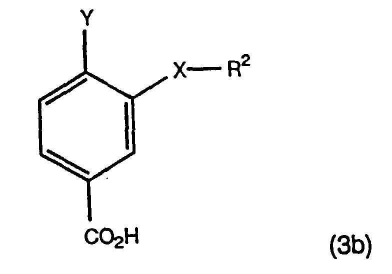 or an active derivative thereof with an amine R⁴(CH₂)ₙNHR³.

## Claims (Claims for the following Contracting State(s): IE, MC)

1. A compound of formula (1) 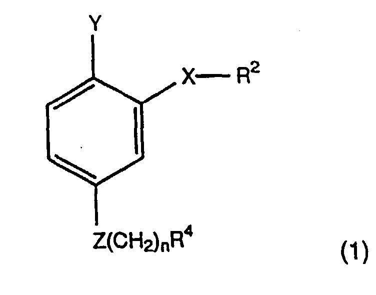 wherein
Y is a group -OR¹ where R¹ is an optionally substituted alkyl group;
R² is an optionally substituted cycloalkyl or polycycloalkyl group;
Z is a group -N(R³)CO- or -CON(R³)- where R³ is a hydrogen atom or an alkyl, aryl or aralkyl group;
R⁴ is an optionally substituted aryl or optionally substituted heteroaryl group;
X is -O-, -S-, -CH₂- or -N(R⁵)-, where R⁵ is a hydrogen atom or an alkyl group;
n is zero or an integer of value 1, 2, or 3; and the salts, solvates and hydrates thereof.

2. A compound according to claim 1 wherein Z is a -N(R³)CO- group.

3. A compound according to claim 2 wherein Z is a -NHCO- group.

4. A compound according to claim 1 wherein Z is a -CON(R³)- group.

5. A compound according to claim 4 wherein Z is a -CONH- group.

6. A compound according to any preceding claim where R¹ is an optionally substituted straight or branched C₁₋₃ alkyl group.

7. A compound according to claim 6 wherein R⁷ is a straight or branched C₁₋₃ alkyl group optionally substituted by one or more halogen atoms.

8. A compound according to claim 7 where R¹ is a methyl group.

9. A compound according to any one of claims 1 to 8 where n is zero or an integer of value 1.

10. A compound according to claim 9 where n is zero.

11. A compound according to any one of claims 1 to 10 where R² is an optionally substituted C₃₋₈ cycloalkyl group.

12. A compound according to claim 11 where R² is a cyclobutyl, cyclopentyl or cyclohexyl group optionally substituted by one, two or three halogen atoms, C₁₋₆ alkyl or C₁₋₆ alkoxy groups.

13. A compound according to claim 12 where R² is a cyclopentyl group.

14. A compound a according to any one of claims 1 to 13 wherein R⁴ is an optionally substituted phenyl or pyridinyl group.

15. A compound according to claim 14 wherein R⁴ is an optionally substituted 4-pyridinyl group.

16. A compound according to any of the preceding claims wherein X is -O-.

17. 3-Cyclopentyloxy-4-methyloxy-N-(2-nitrobenzoyl)aniline;
3-Cyclopentyloxy-4-methyloxy-N-(3-nitrobenzoyl)aniline;
N-Phenyl-3-cyclopentyloxy-4-methyloxybenzamide;
N-(2-Nitrophenyl)-3-cyclopentyloxy-4-methoyloxybenzamide;
N-Benzyl-3-cyclopentyloxy-4-methyloxybenzamide;
and the salts, solvates, and hydrates thereof.

18. A pharmaceutical composition comprising a compound according to any one of claims 1 to 17 and a pharmaceutically acceptable diluent, carrier or excipient.

19. A process for the preparation of a compound of formula (1) 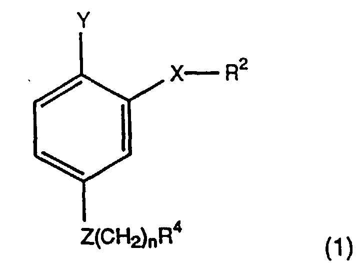 wherein
Y is a group -OR¹ where R¹ is an optionally substituted alkyl group;
R² is an optionally substituted cycloalkyl or polycycloalkyl group;
Z is a group -N(R³)CO- or -CON(R³)- where R³ is a hydrogen atom or an alkyl, aryl or aralkyl group;
R⁴ is an optionally substituted aryl or optionally substituted heteroaryl group;
X is -O-, -S-, -CH₂- or -N(R⁵)-, where R⁵ is a hydrogen atom or an alkyl group;
n is zero or an integer of value 1, 2, or 3; and the salts, solvates and hydrates thereof;
which comprises either (a) coupling an amine of formula (3a) 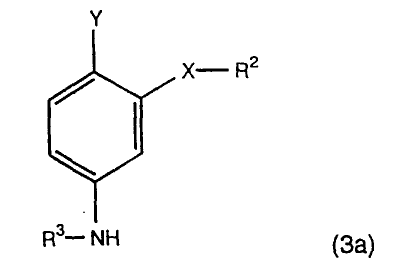 wherein
Y, R², R³ and X are as defined for formula (1), with an acid R⁴(CH₂)ₙCO₂H, where R⁴ and n are as defined for formula (1), or an active derivative thereof or (b) by coupling an acid of formula (3b) 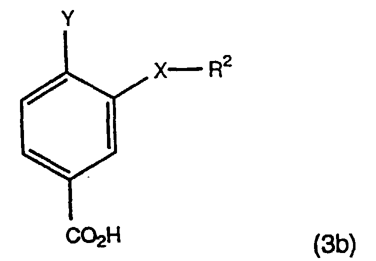 or an active derivative thereof with an amine R⁴(CH₂)ₙNHR³.

## Patentansprüche (Patentansprüche für folgende(n) Vertragsstaat(en): AT, BE, CH, LI, DE, DK, ES, FR, GB, GR, IT, LU, NL, PT, SE)

1. Verbindung der Formel (1) 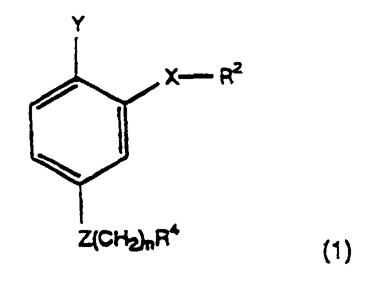 worin
Y eine Gruppe -OR¹ darstellt, wobei R¹ eine gegebenenfalls substituierte Alkylgruppe darstellt;
R² eine gegebenenfalls substituierte Cycloalkyl- oder Polycycloalkylgruppe darstellt;
Z eine Gruppe -N(R³)CO- oder -CON(R³)- darstellt, wobei R³ ein Wasserstoffatom oder eine Alkyl-, Aryl- oder Aralkylgruppe darstellt;
R⁴ eine gegebenenfalls substituierte Aryl- oder gegebenenfalls substituierte Heteroarylgruppe darstellt;
X -O-, -S-, -CH₂- oder -N(R⁵)- darstellt, worin R⁵ ein Wasserstoffatom oder eine Alkylgruppe darstellt;
n null oder eine ganze Zahl im Wert von 1, 2 oder 3 ist;
mit der Maßgabe, daß, wenn:
Z -CONH- darstellt
n null ist;
X -O- darstellt;
R¹ eine unsubstituierte C₁₋₄-Alkylgruppe darstellt und
R² eine unsubstituierte Cycloalkyl- oder Polycycloalkylgruppe darstellt;
dann:
R⁴ eine Phenyl-, Naphthyl- oder Heteroarylgruppe, substituiert mit einem oder mehreren Substituenten, ausgewählt aus C₂₋₆-Alkylendioxy-, C₅₋₇-Cycloalkoxy-, Carboxyl-(-CO₂H), -CO₂R₇ [worin R₇ eine C₆₋₁₂-Aryl-C₁₋₃-alkyl- oder C₆₋₁₂-Arylgruppe, jedoch kein Phenyl oder Naphthyl darstellt], -SO₃H, Sulfonylamino- (-NHSO₂H), C₁₋₆-Alkylsulfonylamino- oder C₁₋₆-Dialkylsulfonylaminogruppen, darstellt
und die Salze, Solvate und Hydrate davon.

2. Verbindung nach Anspruch 1, worin Z eine Gruppe -N(R³)CO- darstellt.

3. Verbindung nach Anspruch 2, worin Z eine Gruppe -NHCO- darstellt.

4. Verbindung nach Anspruch 1, worin Z eine Gruppe -CON(R³)- darstellt.

5. Verbindung nach Anspruch 4, worin Z eine Gruppe -CONH- darstellt.

6. Verbindung nach einem vorangehenden Anspruch, worin R¹ eine gegebenenfalls substituierte geradkettige oder verzweigte C₁₋₃-Alkylgruppe darstellt.

7. Verbindung nach Anspruch 6, worin R₇ eine geradkettige oder verzweigte C₁₋₃-Alkylgruppe, gegebenenfalls substituiert mit einem oder mehreren Halogenatomen, darstellt.

8. Verbindung nach Anspruch 7, worin R¹ eine Methylgruppe darstellt.

9. Verbindung nach einem der Ansprüche 1 bis 8, worin n null oder eine ganze Zahl mit dem Wert 1 ist.

10. Verbindung nach Anspruch 9, worin n null ist.

11. Verbindung nach einem der Ansprüche 1 bis 10, worin R² eine gegebenenfalls substituierte C₃₋₈-Cycloalkylgruppe darstellt.

12. Verbindung nach Anspruch 11, worin R² eine Cyclobutyl-, Cyclopentyl- oder Cyclohexylgruppe, gegebenenfalls substituiert mit einem, zwei oder drei Halogenatomen, C₁₋₆-Alkyl- oder C₁₋₆-Alkoxygruppen, darstellt.

13. Verbindung nach Anspruch 12, worin R² eine Cyclopentylgruppe darstellt.

14. Verbindung nach einem der Ansprüche 1 bis 13, worin R⁴ eine gegebenenfalls substituierte Phenyl- oder Pyridinylgruppe darstellt.

15. Verbindung nach Anspruch 14, worin R⁴ eine gegebenenfalls substituierte 4-Pyridinylgruppe darstellt.

16. Verbindung nach einem der vorangehenden Ansprüche, worin X -O- darstellt.

17. Pharmazeutische Zusammensetzung, umfassend eine Verbindung nach einem der Ansprüche 1 bis 16 und ein pharmazeutisch verträgliches Verdünnungsmittel, einen pharmazeutisch verträglichen Träger oder einen pharmazeutisch verträglichen Exzipienten.

18. Verfahren zur Herstellung einer Verbindung der wie in Anspruch 1 definierten Formel (1), umfassend entweder (a) Kuppeln eines Amins der Formel (3a) 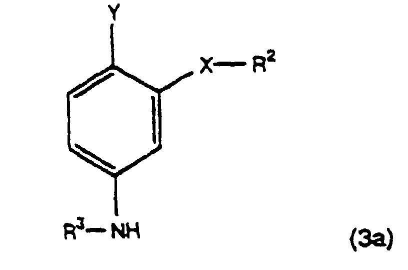 worin
Y, R², R³ und X wie für Formel (1) definiert sind, mit einer Säure R⁴(CH₂)ₙCO₂H, worin R⁴ und n wie für Formel (1) definiert sind oder einem aktiven Derivat davon, oder (b) Kuppeln einer Säure der Formel (3b) 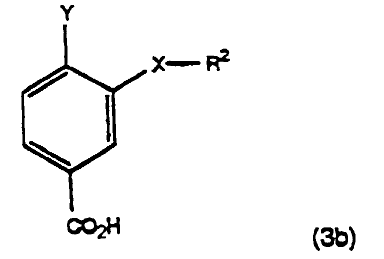 oder eines aktiven Derivats davon mit einem Amin R⁴(CH₂)ₙNHR³.

## Patentansprüche (Patentansprüche für folgende(n) Vertragsstaat(en): IE, MC)

1. Verbindung der Formel (1) 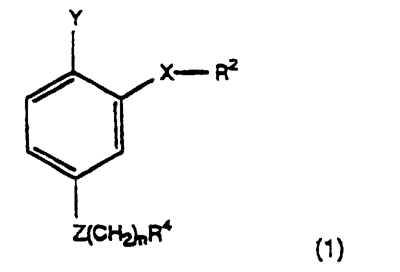 worin
Y eine Gruppe -OR¹ darstellt, worin R¹ eine gegebenenfalls substituierte Alkylgruppe darstellt;
R² eine gegebenenfalls substituierte Cycloalkyl- oder Polycycloalkylgruppe darstellt;
Z eine Gruppe -N(R³)CO- oder -CON(R³)- darstellt, worin R³ ein Wasserstoffatom oder eine Alkyl-, Aryl- oder Aralkylgruppe darstellt;
R⁴ eine gegebenenfalls substituierte Aryl- oder gegebenenfalls substituierte Heteroarylgruppe darstellt;
X -O-, -S-, -CH₂- oder -N(R⁵)- darstellt, worin R⁵ ein Wasserstoffatom oder eine Alkylgruppe darstellt;
n null oder eine ganze Zahl im Wert von 1, 2 oder 3 ist; und die Salze, Solvate und Hydrate davon.

2. Verbindung nach Anspruch 1, worin Z eine Gruppe -N(R³)CO- darstellt.

3. Verbindung nach Anspruch 2, worin Z eine Gruppe -NHCO- darstellt.

4. Verbindung nach Anspruch 1, worin Z eine Gruppe -CON(R³)- darstellt.

5. Verbindung nach Anspruch 4, worin Z eine Gruppe -CONH- darstellt.

6. Verbindung nach einem vorangehenden Anspruch, worin R¹ eine gegebenenfalls substituierte geradkettige oder verzweigte C₁₋₃-Alkylgruppe darstellt.

7. Verbindung nach Anspruch 6, worin R₇ eine geradkettige oder verzweigte C₁₋₃-Alkylgruppe, gegebenenfalls substituiert mit einem oder mehreren Halogenatomen, darstellt.

8. Verbindung nach Anspruch 7, worin R¹ eine Methylgruppe darstellt.

9. Verbindung nach einem der Ansprüche 1 bis 8, worin n null oder eine ganze Zahl mit dem Wert 1 ist.

10. Verbindung nach Anspruch 9, worin n null ist.

11. Verbindung nach einem der Ansprüche 1 bis 10, worin R² eine gegebenenfalls substituierte C₃₋₈-Cycloalkylgruppe darstellt.

12. Verbindung nach Anspruch 11, worin R² eine Cyclobutyl-, Cyclopentyl- oder Cyclohexylgruppe, gegebenenfalls substituiert mit einem, zwei oder drei Halogenatomen, C₁₋₆-Alkyl- oder C₁₋₆-Alkoxygruppen, darstellt.

13. Verbindung nach Anspruch 12, worin R² eine Cyclopentylgruppe darstellt.

14. Verbindung nach einem der Ansprüche 1 bis 13, worin R⁴ eine gegebenenfalls substituierte Phenyl- oder Pyridinylgruppe darstellt.

15. Verbindung nach Anspruch 14, worin R⁴ eine gegebenenfalls substituierte 4-Pyridinylgruppe darstellt.

16. Verbindung nach einem der vorangehenden Ansprüche, worin X -O- darstellt.

17. 3-Cyclopentyloxy-4-methyloxy-N-(2-nitrobenzoyl)anilin; 3-Cyclopentyloxy-4-methyloxy-N-(3-nitrobenzoyl)anilin; N-Phenyl-3-cyclopentyloxy-4-methyloxybenzamid; N-(2-Nitrophenyl)-3-cyclopentyloxy-4-methoyloxybenzamid; N-Benzyl-3-cyclopentyloxy-4-methyloxybenzamid; und die Salze, Solvate und Hydrate davon.

18. Pharmazeutische Zusammensetzung, umfassend eine Verbindung nach einem der Ansprüche 1 bis 17 und ein pharmazeutisch verträgliches Verdünnungsmittel, einen pharmazeutisch verträglichen Träger oder einen pharmazeutisch verträglichen Exzipienten.

19. Verfahren zur Herstellung einer Verbindung der Formel (1) 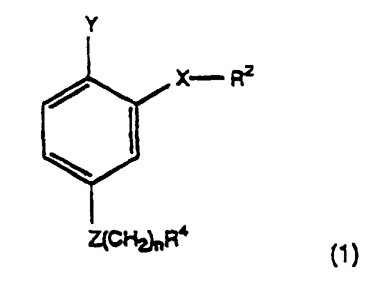 worin
Y eine Gruppe -OR¹ darstellt, worin R¹ eine gegebenenfalls substituierte Alkylgruppe darstellt;
R² eine gegebenenfalls substituierte Cycloalkyl- oder Polycycloalkylgruppe darstellt;
Z eine Gruppe -N(R³)CO- oder -CON(R³)- darstellt, worin R³ ein Wasserstoffatom oder eine Alkyl-, Aryl- oder Aralkylgruppe darstellt;
R⁴ eine gegebenenfalls substituierte Aryl- oder gegebenenfalls substituierte Heteroarylgruppe darstellt;
X -O-, -S-, -CH₂- oder -N(R⁵)- darstellt, worin R⁵ ein Wasserstoffatom oder eine Alkylgruppe darstellt;
n null oder eine ganze Zahl im Wert von 1, 2 oder 3 ist; und den Salzen, Solvaten und Hydraten davon;
umfassend entweder (a) Kuppeln eines Amins der Formel (3a) 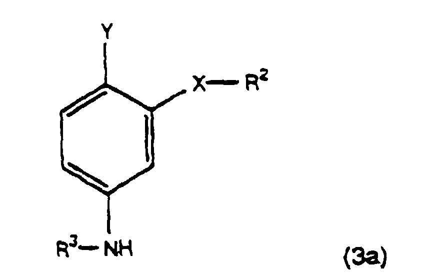 worin
Y, R², R³ und X wie für Formel (1) definiert sind, mit einer Säure R⁴(CH₂)ₙCO₂H, worin R⁴ und n wie für Formel (1) definiert sind oder einem aktiven Derivat davon, oder (b) Kuppeln einer Säure der Formel (3b) 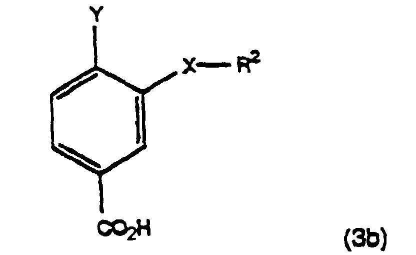 oder eines aktiven Derivats davon mit einem Amin R⁴(CH₂)ₙNHR³.

## Revendications (Revendications pour l'(les) Etat(s) contractant(s) suivant(s): AT, BE, CH, LI, DE, DK, ES, FR, GB, GR, IT, LU, NL, PT, SE)

1. Composé de formule (1) 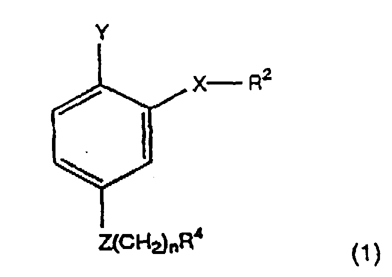 où
Y est un groupe -OR¹ où R¹ est un groupe alkyle facultativement substitué ;
R² est un groupe cycloalkyle ou polycycloalkyle facultativement substitué ;
Z est un groupe -N(R³)CO- ou -CON(R³)- où R³ est un atome d'hydrogène ou un groupe alkyle, aryle ou aralkyle ;
R⁴ est un groupe aryle facultativement substitué ou hétéroaryle facultativement substitué ;
X est -O-, -S-, -CH₂- ou -N(R⁵)-, où R⁵ est un atome d'hydrogène ou un groupe alkyle ;
n est zéro ou un nombre entier de valeur 1, 2 ou 3 ;
à condition que si :
Z est -CONH- ;
n est zéro ;
X est -O- ;
R¹ est un groupe alkyle en C₁-C₄ non substitué ; et
R² est un groupe cycloalkyle ou polycycloalkyle non substitué ;
alors :
R⁴ est soit un groupe phényle, naphtyle ou hétéroaryle substitué par un ou plusieurs substituants choisis parmi les groupes alkylènedioxy en C₂-C₆, cycloalcoxy en C₅-C₇, carboxyle (-CO₂H), -CO₂R₇ [où R₇ est un groupe (aryle en C₆-C₁₂)-(alkyle en C₁-C₃) ou aryle en C₆-C₁₂, mais n'est pas un groupe phényle ou naphtyle], -SO₃H, sulfonylamino (-NHSO₂H), (alkyle en C₁-C₆)sulfonylamino ou (dialkyle en C₁-C₆)sulfonylamino ;
et ses sels, solvats et hydrates.

2. Composé selon la revendication 1, dans lequel Z est un groupe -N(R³)CO-.

3. Composé selon la revendication 2, dans lequel Z est un groupe -NHCO-.

4. Composé selon la revendication 1, dans lequel Z est un groupe -CON(R³)-.

5. Composé selon la revendication 4, dans lequel Z est un groupe -CONH-.

6. Composé selon l'une quelconque des revendications précédentes, dans lequel R¹ est un groupe alkyle en C₁-C₃ linéaire ou ramifié, facultativement substitué.

7. Composé selon la revendication 6, dans lequel R⁷ est un groupe alkyle en C₁-C₃ linéaire ou ramifié, facultativement substitué par un ou plusieurs atomes d'halogène.

8. Composé selon la revendication 7, dans lequel R¹ est un groupe méthyle.

9. Composé selon l'une quelconque des revendications 1 à 8, dans lequel n est zéro ou un nombre entier de valeur 1.

10. Composé selon la revendication 9, dans lequel n est zéro.

11. Composé selon l'une quelconque des revendications 1 à 10, dans lequel R² est un groupe cycloalkyle en C₃-C₈ facultativement substitué.

12. Composé selon la revendication 11, dans lequel R² est un groupe cyclobutyle, cyclopentyle ou cyclohexyle facultativement substitué par un, deux ou trois atomes d'halogène, groupes alkyle en C₁-C₆ ou groupes alcoxy en C₁-C₆.

13. Composé selon la revendication 12, dans lequel R² est un groupe cyclopentyle.

14. Composé selon l'une quelconque des revendications 1 à 13, dans lequel R⁴ est un groupe phényle ou pyridinyle facultativement substitué.

15. Composé selon la revendication 14, dans lequel R⁴ est un groupe 4-pyridinyle facultativement substitué.

16. Composé selon l'une quelconque des revendications précédentes, dans lequel X est -O-.

17. Composition pharmaceutique comprenant un composé selon l'une quelconque des revendications 1 à 16 et un diluant, support ou excipient pharmaceutiquement acceptable.

18. Procédé pour la préparation d'un composé de formule (1) tel que défini dans la revendication 1, qui comprend soit (a) le couplage d'une amine de formule (3a) 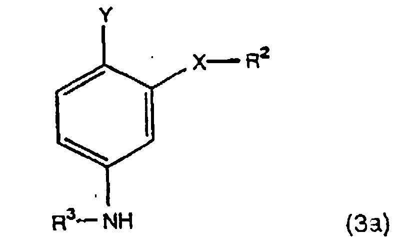 où Y, R², R³ et X sont tels que définis pour la formule (1), avec un acide R⁴(CH₂)ₙCO₂H, où R⁴ et n sont tels que définis pour la formule (1), ou un dérivé actif de celui-ci, soit (b) le couplage d'un acide de formule (3b) 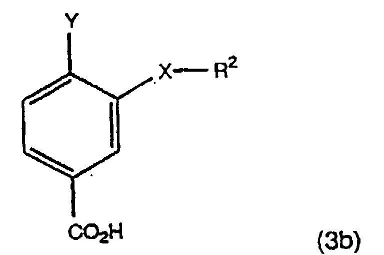 ou d'un dérivé actif de celui-ci, avec une amine R⁴(CH₂)ₙNHR³.

## Revendications (Revendications pour l'(les) Etat(s) contractant(s) suivant(s): IE, MC)

1. Composé de formule (1) 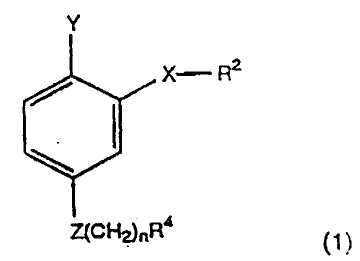 où
Y est un groupe -OR¹ où R¹ est un groupe alkyle facultativement substitué ;
R² est un groupe cycloalkyle ou polycycloalkyle facultativement substitué ;
Z est un groupe -N(R³)CO- ou -CON(R³)- où R³ est un atome d'hydrogène ou un groupe alkyle, aryle ou aralkyle ;
R⁴ est un groupe aryle facultativement substitué ou hétéroaryle facultativement substitué ;
X est -O-, -S-, -CH₂- ou -N(R⁵)-, où R⁵ est un atome d'hydrogène ou un groupe alkyle ;
n est zéro ou un nombre entier de valeur 1, 2 ou 3 ; et ses sels, solvats et hydrates.

2. Composé selon la revendication 1, dans lequel Z est un groupe -N(R³)CO-.

3. Composé selon la revendication 2, dans lequel Z est un groupe -NHCO-.

4. Composé selon la revendication 1, dans lequel Z est un groupe -CON(R³)-.

5. Composé selon la revendication 4, dans lequel Z est un groupe -CONH-.

6. Composé selon l'une quelconque des revendications précédentes, dans lequel R¹ est un groupe alkyle en C₁-C₃ linéaire ou ramifié, facultativement substitué.

7. Composé selon la revendication 6, dans lequel R⁷ est un groupe alkyle en C₁-C₃ linéaire ou ramifié, facultativement substitué par un ou plusieurs atomes d'halogène.

8. Composé selon la revendication 7, dans lequel R¹ est un groupe méthyle.

9. Composé selon l'une quelconque des revendications 1 à 8, dans lequel n est zéro ou un nombre entier de valeur 1.

10. Composé selon la revendication 9, dans lequel n est zéro.

11. Composé selon l'une quelconque des revendications 1 à 10, dans lequel R² est un groupe cycloalkyle en C₃-C₈ facultativement substitué.

12. Composé selon la revendication 11, dans lequel R² est un groupe cyclobutyle, cyclopentyle ou cyclohexyle facultativement substitué par un, deux ou trois atomes d'halogène, groupes alkyle en C₁-C₆ ou groupes alcoxy en C₁-C₆.

13. Composé selon la revendication 12, dans lequel R² est un groupe cyclopentyle.

14. Composé selon l'une quelconque des revendications 1 à 13, dans lequel R⁴ est un groupe phényle ou pyridinyle facultativement substitué.

15. Composé selon la revendication 14, dans lequel R⁴ est un groupe 4-pyridinyle facultativement substitué.

16. Composé selon l'une quelconque des revendications précédentes, dans lequel X est -O-.

17. 3-cyclopentyloxy-4-méthyloxy-N-(2-nitrobenzoyl)aniline ; 3-cyclopentyloxy-4-méthyloxy-N-(3-nitrobenzoyl)aniline ; N-phényl-3-cyclopentyloxy-4-méthyloxybenzamide ; N-(2-nitrophényl)-3-cyclopentyloxy-4-méthyloxybenzamide ; N-benzyl-3-cyclopentyloxy-4-méthyloxybenzamide ; et leurs sels, solvats et hydrates.

18. Composition pharmaceutique comprenant un composé selon l'une quelconque des revendications 1 à 17 et un diluant, support ou excipient pharmaceutiquement acceptable.

19. Procédé pour la préparation d'un composé de formule (1) 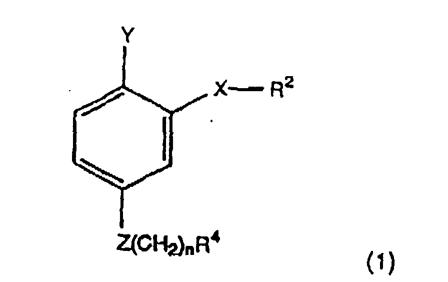 où
Y est un groupe -OR¹ où R¹ est un groupe alkyle facultativement substitué ;
R² est un groupe cycloalkyle ou polycycloalkyle facultativement substitué ;
Z est un groupe -N(R³)CO- ou -CON(R³)- où R³ est un atome d'hydrogène ou un groupe alkyle, aryle ou aralkyle ;
R⁴ est un groupe aryle facultativement substitué ou hétéroaryle facultativement substitué ;
X est -O-, -S-, -CH₂- ou -N(R⁵)-, où R⁵ est un atome d'hydrogène ou un groupe alkyle ;
n est zéro ou un nombre entier de valeur 1, 2 ou 3 ; et de ses sels, solvats et hydrates ;
qui comprend soit (a) le couplage d'une amine de formule (3a) 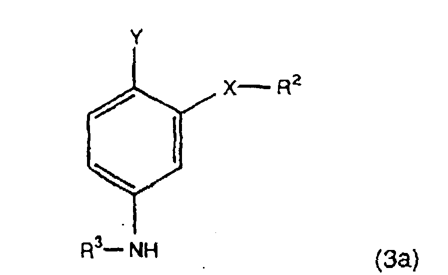 où Y, R², R³ et X sont tels que définis pour la formule (1), avec un acide R⁴(CH₂)ₙCO₂H, où R⁴ et n sont tels que définis pour la formule (1), ou un dérivé actif de celui-ci, soit (b) le couplage d'un acide de formule (3b) 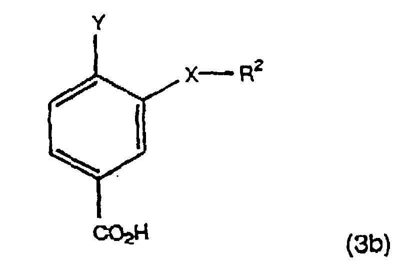 ou d'un dérivé actif de celui-ci, avec une amine R⁴(CH₂)ₙNHR³.
